# EUROPÄISCHE PATENTSCHRIFT

(11) **EP 3 616 756 B1**
(45) Veröffentlichungstag und Bekanntmachung des Hinweises auf die Patenterteilung: **09.03.2022**
(21) Anmeldenummer: 19191424.1
(22) Anmeldetag: 13.08.2019
(51) Int. Cl.: A61Q 7/00, A61K 8/67, A61K 8/49, A61K 8/44, A61K 8/37, A61K 8/63

(54) **MITTEL ZUR FÖRDERUNG VON HAARWUCHS, ANWENDUNG DES HAARWUCHSMITTELS UND VERFAHREN ZU SEINER HERSTELLUNG**
COMPOSITION FOR PROMOTING HAIR GROWTH, USE OF THE HAIR GROWTH AGENT AND METHOD FOR PRODUCING THE SAME
COMPOSITION FAVORISANT LA POUSSE DES CHEVEUX, UTILISATION DE L'AGENT DE POUSSE DES CHEVEUX ET SON PROCEDE DE PRODUCTION

(30) Priorität: 31.08.2018 DE 102018121283
(43) Veröffentlichungstag der Anmeldung: 04.03.2020
(73) Patentinhaber: Müller-Kähmann, Dieter Klaus, 66450 Neunkirchen (DE)
(72) Erfinder: Müller-Kähmann, Dieter Klaus, 66450 Neunkirchen (DE)
(74) Vertreter: Patentanwälte Bernhardt / Wolff Partnerschaft mbB

(56) Entgegenhaltungen:
- EP-A2- 0 489 581
- WO-A1-2018/049174
- DE-A1- 1 617 477
- DE-A1- 10 050 669
- DE-T2- 69 308 928
- US-A1- 2002 028 257
- US-A1- 2013 309 282

## Beschreibung

Die Erfindung betrifft ein Mittel zur Förderung von Haarwuchs, insbesondere des Kopfhaars von Männern, das einen durchblutungsfördernden Stoff, einen blutdrucksenken-den Stoff, einen Stoff zur Förderung des Stoffwechsels in der Haut und einen entzündungshemmenden Stoff enthält.

Haarausfall im Bereich des Kopfhaars tritt insbesondere bei Männern in zunehmendem Alter auf und wird von den Betroffenen oftmals als Problem empfunden. Durch Benutzung sind diverse Haarwuchsmittel bekannt. Allerdings wirken die bekannten Haarwuchsmittel nicht oder nicht ausreichend.

Ein Mittel zur Förderung von Haarwuchs der obengenannten Art geht aus der WO 2018/049174 A1 hervor, das Verapamil, Minoxidil und Diclofenac-NSAID enthält.

DE 100 50 669 A1 beschreibt ein Haarwuchsförderungsmittel, das den Stoff Angelica chinensis, den Stoff Ganoderma lucidum, Astragalus, roten Ginseng und Meeralgen enthält.

In der EP 0 489 581 A2 ist ein Haarwuchsförderungsmittel beschrieben, das Minoxidil, Benzylnicotinat und Zitronensäure enthält.

Weitere Veröffentlichungen, die Haarwuchsmittel betreffen sind DE 693 08 928 T2, DE 16 17 477 A1, US 2002/0028257 A1 und US 2013/309282 A1.

Der Erfindung liegt die Aufgabe zugrunde, ein Haarwuchsmittel zu schaffen, das Haarverlust stärker reduziert und Haarwachstum besser fördert als die bekannten Haarwuchsmittel.

Erfindungsgemäß wird diese Aufgabe dadurch gelöst, dass der Stoff zur Förderung des Stoffwechsels in der Haut Biotin und/oder Tyrosin ist und das Mittel Cayennepfeffer als durchblutungsfördernden Stoff in einer Konzentration von 2 bis 10 mg/l enthält.

Das erfindungsgemäße Haarwuchsmittel reduziert den Haarausfall und fördert das Haarwachstum durch eine verbesserte Durchblutung ungenügend versorgter Kopfhautpartien. Dadurch können zum einen körpereigene Aufbau- und Abwehrstoffe besser zu ermüdeten oder unterversorgten Haarwurzeln transportiert werden, zum anderen können Stoffwechselprodukte, die schädlich für die Haarwurzeln sind, schneller entfernt werden. Das Haarwuchsmittel erhöht die Mikrozirkulation in der Kopfhaut und damit deren Nährstoffversorgung, aktiviert die Haarwurzeln und die Zellteilung, verbessert dadurch das Haarwachstum und verlängert die Wachstumsphase der Haare. Darüber hinaus wird die Kopfhaut gegen für die Haarwurzeln schädliche Auswirkungen von Testosteron geschützt.

Überraschend hat sich gezeigt, dass das Haarwuchsmittel aufgrund der gefundenen Stoffkombinationen wesentlich besser wirkt als die bekannten Haarwuchsmittel.

Der durchblutungsfördernde Stoff kann ein Wirkstoff aus der Gruppe der Rubefazienzien und der Nikotinsäureester sein. Bevorzugt wird Benzylnicotinat verwendet, das sich besonders gut zur direkten Anwendung auf der Haut eignet.

Der Cayennepfeffer reizt die Kopfhaut und verursacht eine Öffnung der Blutgefäße, die ebenfalls die Mikrozirkulation und die Versorgung der Kopfhautzellen und der Haarwurzeln fördert.

In einer Ausführungsform der Erfindung enthält das Mittel als den blutdrucksenkenden Stoff Minoxidil. Dieses Antihypertonikum erhöht die Öffnungswahrscheinlichkeit ATPabhängiger Kaliumkanäle. Dadurch nimmt das Membranruhepotential zu und der Einstrom von Kalzium in die Kaliumkanäle wird verringert. Dies führt vor allem in Arteriolen zur Verringerung des Tonus der glatten Gefäßmuskulatur und dadurch zur Blutdrucksenkung.

Zweckmäßigerweise enthält das Haarwuchsmittel zur Förderung des Stoffwechsels in der Haut Biotin und/oder Tyrosin. Das Biotin wirkt sich positiv auf den Stoffwechsel der Keratinozyten aus, sobald es in die tieferen Haarwurzeln transportiert wird. Durch das Tyrosin wird der Dopaminspiegel in der Kopfhaut erhöht. Dies hat zur Folge, dass die Haarwurzeln besser wachsen und sich die Haare besser aufbauen können.

In einer besonders bevorzugten Ausführungsform der Erfindung enthält das Mittel als den entzündungshemmenden Stoff Hydroxyethylsalicylat, Natriumbituminosulfonat und/oder Prednisolon.

Das Hydroxyethylsalicylat wirkt einerseits entzündungshemmend und bei vorhandenen Entzündungen abschwellend. Darüber hinaus fördert es die Mikrozirkulation und damit die Durchblutung der Mikrogefäße, insbesondere der Kapillaren.

Das Natriumbituminosulfonat, das bevorzugt als helles Natriumbituminosulfonat verwendet wird, wirkt juckreizstillend, entzündungshemmend, anti-exzematös, antiseborrhoisch, bakteriostatisch und darüber hinaus antimyzetisch.

Das Prednisolon wirkt antiinflammatorisch und verhindert die Ausbildung von Entzündungen, die das Haarwachstum stören.

In einer weiteren Ausgestaltung der Erfindung enthält das Haarwuchsmittel Estradiolbenzoat. Durch dieses Estrogen wird die Wirkung von Testosteron blockiert, die für das Haarwachstum schädlich ist, insbesondere da das Testosteron in der Kopfhaut zu einer Verfettung der Haarwurzeln führt.

Als Träger weist das Mittel zweckmäßigerweise ein Lösungsmittel auf. Das Lösungsmittel kann wässrig, alkoholisch oder als alkoholisch-wässrig sein. Als besonders geeignet hat sich die Benutzung von Isopropanol, vorzugsweise 30 bis 70 %igem Isopropanol, erwiesen. Darüber hinaus kann das Mittel zur Stabilisierung der Lösung Propylenglycol enthalten.

Die Wirkung des Haarwuchsmittels hat sich als besonders wirksam erwiesen, wenn es die nachfolgend genannten Stoffe in folgenden Konzentrationen in mg/l enthält:

| | |
|---|---|
| - Benzylnicotinat: | 100,0 bis 200,0, vorzugsweise 140,0 bis 160,0 |
| - Minoxidil: | 30,0 bis 200,0, vorzugsweise 40,0 bis 60,0 |
| - Biotin: | 0,1 bis 0,8, vorzugsweise 0,15 bis 0,25 |
| - Tyrosin: | 300,0 bis 800,0, vorzugsweise 350,0 bis 130,0 |
| - Hydroxyethylsalicylat: | 100,0 bis 200,0, vorzugsweise 110,0 bis 130,0 |
| - Natriumbituminosulfonat: | 3,0 bis 15,0, vorzugsweise 4,0 bis 5,0 |

In einer weiteren Ausgestaltung der Erfindung enthält das Mittel Cayennepfeffer, insbesondere Cayennepfeffer-Pflanzen-Extrakt, in einer Konzentration von 2 bis 10 mg/l, vorzugsweise 3 bis 8 mg/l. Zweckmäßigerweise weist das Mittel den Cayennepfeffer in Form einer Tinktur auf. Eine solche Tinktur kann zur Herstellung des Haarwuchsmittels als Bestandteil beigemischt werden.

Als besonders geeignet für die Herstellung des Haarwuchsmittels hat sich die Verwendung von Tinctura Capsici, insbesondere Tinctura Capsici Normata (standardisierte Cayennepfeffertinktur) erwiesen. Das Haarwuchsmittel enthält in der bevorzugten Ausführungsform der Erfindung 40 bis 60 mg, vorzugsweise 45 bis 55 mg, der Tinctura Capsici Normata.

Zweckmäßigerweise enthält das Haarwuchsmittel das Estradiolbenzoat in einer Konzentration von 2 bis 10 mg/l, vorzugsweise 4 bis 6 mg/l.

Das Prednisolon wird in einer Ausführungsform der Erfindung in dem Mittel in einer Konzentration von 100 bis 300 mg/l, vorzugsweise 150 bis 250 mg/l, vorgesehen.

Das erfindungsgemäße Haarwuchsmittel eignet sich neben der allgemeinen Förderung von Haarwuchs, insbesondere im Bereich des Kopfhaars von Männern, sowohl zur prophylaktischen Behandlung von Haarausfall als auch zur Behandlung von akutem Haarausfall. Das Mittel wird zweckmäßigerweise auf die Kopfhaut aufgetragen und kann in die Kopfhaut einmassiert werden.

Zur Herstellung des Haarwuchsmittels werden der durchblutungsfördernde Stoff, der blutdrucksenkende Stoff, der Stoff zur Förderung des Stoffwechsels in der Haut und der entzündungshemmende Stoff in dem Lösungsmittel, vorzugsweise einem wässrigen, einem alkoholischen oder einem wässrig-alkoholischen Lösungsmittel gelöst und die erhaltene Lösung gerührt. Wie oben bereits erläutert, eignet sich als Lösungsmittel insbesondere Isopropanol, wobei zur Stabilisierung der Lösung vorzugsweise Propylenglycol hinzugegeben wird.

### Beispiel:

Es wird ein erfindungsgemäßes Haarwuchsmittel hergestellt, indem die folgenden Stoffe miteinander vermischt werden:

| | | |
|---|---|---|
| - Isopropanol (50 %ig): | 400,0 | g |
| - Hydroxyethylsalicylat: | 120,0 | mg |
| - Benzylnicotinat: | 150,0 | mg |
| - Minoxidil: | 50,0 | mg |
| - Natriumbituminosulfonat (hell): | 5,0 | mg |
| - Propylenglycol: | 5,0 | mg |
| - Tinctura Capsici Normata (85 %): | 50,0 | mg |
| - Tyrosin: | 400,0 | mg |
| - Biotin: | 0,2 | mg |
| - Estradiolbenzoat: | 0,005 | g |
| - Prednisolon: | 0,2 | g |

Das erhaltene Haarwuchsmittel wurde von 10 Männern, die unter fortschreitendem Haarausfall am Kopf litten, während einem Zeitraum von einem Jahr einmal täglich in die Kopfhaut einmassiert und dort wirken gelassen. Bei den Männern kam es nicht zu weiterem Haarverlust. Das Haar ist wieder dichter geworden.

## Patentansprüche

1. Mittel zur Förderung von Haarwuchs, insbesondere des Kopfhaars von Männern, das einen durchblutungsfördernden Stoff, einen blutdrucksenkenden Stoff, einen Stoff zur Förderung des Stoffwechsels in der Haut und einen entzündungshemmenden Stoff enthält,
**dadurch gekennzeichnet,**
**dass** der Stoff zur Förderung des Stoffwechsels in der Haut Biotin und/oder Tyrosin ist und das Mittel Cayennepfeffer als durchblutungsfördernden Stoff in einer Konzentration von 2 bis 10 mg/l enthält.

2. Mittel nach Anspruch 1,
**dadurch gekennzeichnet,**
**dass** der Cayennepfeffer ein Cayennepfeffer-Extrakt ist.

3. Mittel nach Anspruch 1 oder 2,
**dadurch gekennzeichnet,**
**dass** der durchblutungsfördernde Stoff Benzylnicotinat aufweist.

4. Mittel nach einem der Ansprüche 1 bis 3,
**dadurch gekennzeichnet,**
**dass** der blutdrucksenkende Stoff Minoxidil ist.

5. Mittel nach einem der Ansprüche 1 bis 4,
**dadurch gekennzeichnet,**
**dass** der entzündungshemmende Stoff ein Stoff ist, der aus der Gruppe bestehend aus Hydroxyethylsalicylat, Natriumbituminosulfonat und Prednisolon ausgewählt ist.

6. Mittel nach einem der Ansprüche 1 bis 5,
**dadurch gekennzeichnet,**
**dass** das Mittel ein wässriges, ein alkoholisches oder ein wässrig-alkoholisches Lösungsmittel, vorzugsweise Isopropanol, enthält.

7. Mittel nach einem der Ansprüche 1 bis 6,
**dadurch gekennzeichnet,**
**dass** das Mittel die nachfolgend genannten Stoffe in folgenden Konzentrationen in mg/l enthält:
| | |
|---|---|
| - Benzylnicotinat: | 100,0 bis 200,0 |
| - Minoxidil: | 30,0 bis 200,0 |
| - Biotin: | 0,1 bis 0,8 |
| - Tyrosin: | 300,0 bis 800,0 |
| - Hydroxyethylsalicylat: | 100,0 bis 200,0 |
| - Natriumbituminosulfonat: | 3,0 bis 15,0 |

8. Mittel nach einem der Ansprüche 1 bis 7,
**dadurch gekennzeichnet,**
**dass** das Mittel Cayennepfeffer in einer Konzentration von 3 bis 8 mg/l enthält.

9. Mittel nach einem der Ansprüche 1 bis 7,
**dadurch gekennzeichnet,**
**dass** das Mittel ein Estrogen, vorzugsweise Estradiolbenzoat, enthält, wobei das Estradiolbenzoat in dem Mittel vorzugsweise in einer Konzentration von 2 bis 10 mg/l, bevorzugt 4 bis 6 mg/l, vorliegt.

10. Mittel nach einem der Ansprüche 1 bis 7,
**dadurch gekennzeichnet,**
**dass** das Mittel Prednisolon enthält, wobei das Prednisolon in dem Mittel vorzugsweise in einer Konzentration von 100 bis 300 mg/l, vorzugsweise 150 bis 250 mg/l, vorliegt.

11. Mittel nach den Ansprüchen 1 bis 10 zur Förderung von Haarwuchs, insbesondere des Kopfhaars von Männern.

12. Mittel nach Anspruch 11 zur prophylaktischen Behandlung von Haarausfall.

13. Mittel nach Anspruch 11 oder 12,
**dadurch gekennzeichnet,**
**dass** das Mittel auf die Kopfhaut aufgetragen wird und vorzugsweise in die Kopfhaut einmassiert wird.

14. Verfahren zur Herstellung eines Mittel zur Förderung von Haarwuchs, insbesondere eines Haarwuchsmittels nach einem der Ansprüche 1 bis 9, wobei in einem Lösungsmittel ein durchblutungsfördernder Stoff, ein blutdrucksenkender Stoff, ein Stoff zur Förderung des Stoffwechsels in der Haut und ein entzündungshemmender Stoff gelöst werden,
**dadurch gekennzeichnet,**
**dass** als der Stoff zur Förderung des Stoffwechsels in der Haut Biotin und/oder Tyrosin gelöst werden und das Mittel Cayennepfeffer als durchblutungsfördernden Stoff in einer Konzentration von 2 bis 10 mg/l enthält.

15. Verfahren nach Anspruch 14,
**dadurch gekennzeichnet,**
**dass** die nachfolgend genannten Stoffe in folgenden Konzentrationen in mg/l in dem Lösungsmittel gelöst werden:
| | |
|---|---|
| - Benzylnicotinat: | 100,0 bis 200,0 |
| - Minoxidil: | 50,0 bis 200,0 |
| - Biotin: | 0,2 bis 0,8 |
| - Tyrosin: | 300,0 bis 800,0 |
| - Hydroxyethylsalicylat: | 100,0 bis 200,0 |
| - Natriumbituminosulfonat: | 3,0 bis 15,0 |

## Claims

1. Composition for promoting hair growth, especially head hair of men, which comprises a circulation-promoting substance, an antihypertensive substance, a substance for promoting metabolism in the skin and an anti-inflammatory substance,
**characterized in that**
the substance for promoting metabolism in the skin is biotin and/or tyrosine and the composition comprises cayenne pepper as circulation-promoting substance at a concentration of 2 to 10 mg/l.

2. Composition according to Claim 1,
**characterized in that**
the cayenne pepper is a cayenne pepper extract.

3. Composition according to Claim 1 or 2, **characterized in that**
the circulation-promoting substance comprises benzyl nicotinate.

4. Composition according to any of Claims 1 to 3, **characterized in that**
the antihypertensive substance is minoxidil.

5. Composition according to any of Claims 1 to 4, **characterized in that**
the anti-inflammatory substance is a substance selected from the group consisting of hydroxyethyl salicylate, sodium bituminosulfonate and prednisolone.

6. Composition according to any of Claims 1 to 5, **characterized in that**
the composition comprises an aqueous, an alcoholic or an aqueous-alcoholic solvent, preferably isopropanol.

7. Composition according to any of Claims 1 to 6, **characterized in that**
the composition comprises the following specified substances at the following concentrations in mg/l:
| | |
|---|---|
| - benzyl nicotinate: | 100.0 to 200.0 |
| - minoxidil: | 30.0 to 200.0 |
| - biotin: | 0.1 to 0.8 |
| - tyrosine: | 300.0 to 800.0 |
| - hydroxyethyl salicylate: | 100.0 to 200.0 |
| - sodium bituminosulfonate: | 3.0 to 15.0 |

8. Composition according to any of Claims 1 to 7, **characterized in that**
the composition comprises cayenne pepper at a concentration of 3 to 8 mg/l.

9. Composition according to any of Claims 1 to 7, **characterized in that**
the composition comprises an oestrogen, preferably oestradiol benzoate, wherein the oestradiol benzoate is present in the composition preferably at a concentration of 2 to 10 mg/l, preferably 4 to 6 mg/l.

10. Composition according to any of Claims 1 to 7, **characterized in that**
the composition comprises prednisolone, wherein the prednisolone is present in the composition preferably at a concentration of 100 to 300 mg/l, preferably 150 to 250 mg/l.

11. Composition according to Claims 1 to 10 for promoting hair growth, especially head hair of men.

12. Composition according to Claim 11 for prophylactic treatment of hair loss.

13. Composition according to Claim 11 or 12, **characterized in that**
the composition is applied to the scalp and is preferably massaged into the scalp.

14. Process for preparing a composition for promoting hair growth, especially a hair growth composition according to any of Claims 1 to 9, wherein a circulation-promoting substance, an antihypertensive substance, a substance for promoting metabolism in the skin and an anti-inflammatory substance are dissolved in a solvent, **characterized in that**
biotin and/or tyrosine are dissolved as the substance for promoting metabolism in the skin and the composition comprises cayenne pepper as circulation-promoting substance at a concentration of 2 to 10 mg/l.

15. Process according to Claim 14,
**characterized in that**
the following specified substances are dissolved in the solvent at the following concentrations in mg/l:
| | |
|---|---|
| - benzyl nicotinate: | 100.0 to 200.0 |
| - minoxidil: | 50.0 to 200.0 |
| - biotin: | 0.2 to 0.8 |
| - tyrosine: | 300.0 to 800.0 |
| - hydroxyethyl salicylate: | 100.0 to 200.0 |
| - sodium bituminosulfonate: | 3.0 to 15.0 |

## Revendications

1. Produit favorisant la pousse des cheveux, en particulier des cheveux de tête chez les hommes, contenant une substance favorisant la circulation sanguine, une substance abaissant la pression sanguine, une substance favorisant le métabolisme dans la peau et une substance anti-inflammatoire,
**caractérisée en ce que**
la substance favorisant le métabolisme dans la peau est la biotine et/ou la tyrosine, et le produit contient du poivre de Cayenne comme substance favorisant la circulation sanguine à une concentration de 2 à 10 mg/l.

2. Produit selon la revendication 1,
**caractérisé en ce que**
le poivre de Cayenne est un extrait de poivre de Cayenne.

3. Produit selon la revendication 1 ou 2,
**caractérisé en ce que**
la substance favorisant la circulation sanguine contient le nicotinate de benzyle.

4. Produit selon l'une des revendications 1 à 3,
**caractérisé en ce que**
la substance abaissant la pression sanguine est le minoxidil.

5. Produit selon l'une des revendications 1 à 4,
**caractérisé en ce que**
la substance anti-inflammatoire est une substance choisie parmi le groupe constitué par le salicylate d'hydroxyéthyle, le bituminosulfonate de sodium et la prednisolone.

6. Produit selon l'une des revendications 1 à 5,
**caractérisé en ce que**
le produit contient un solvant aqueux, alcoolique ou hydro-alcoolique, de préférence de l'isopropanol.

7. Produit selon l'une des revendications 1 à 6,
**caractérisé en ce que**
le produit contient les substances mentionnées ci-après dans les concentrations suivantes en mg/l :
| | |
|---|---|
| - nicotinate de benzyle : | 100,0 à 200,0 |
| - minoxidil : | 30,0 à 200,0 |
| - biotine : | 0,1 à 0,8 |
| - tyrosine : | 300,0 à 800,0 |
| - salicylate d'hydroxyéthyle : | 100,0 à 200,0 |
| - bituminosulfonate de sodium : | 3,0 à 15,0 |

8. Produit selon l'une des revendications 1 à 7,
**caractérisé en ce que**
le produit contient du poivre de Cayenne à une concentration de 3 à 8 mg/l.

9. Produit selon l'une des revendications 1 à 7,
**caractérisé en ce que**
le produit contient un estrogène, de préférence du benzoate d'estradiol, le benzoate d'estradiol étant de préférence présent dans le produit à une concentration de 2 à 10 mg/l, de préférence de 4 à 6 mg/l.

10. Produit selon l'une des revendications 1 à 7,
**caractérisé en ce que**
le produit contient de la prednisolone, de préférence la prednisolone étant présente dans le produit à une concentration de 100 à 300 mg/l, de préférence de 150 à 250 mg/l.

11. Produit selon les revendications 1 à 10 pour favoriser la pousse des cheveux, en particulier des cheveux de tête chez les hommes.

12. Produit selon la revendication 11 pour le traitement prophylactique de la chute des cheveux.

13. Produit selon la revendication 11 ou 12,
**caractérisé en ce que**
le produit est appliqué sur le cuir chevelu et est de préférence massé dans le cuir chevelu.

14. Procédé de préparation d'un produit favorisant la croissance des cheveux, en particulier d'un produit favorisant la croissance des cheveux selon l'une des revendications 1 à 9,
dans lequel
on dissout dans un solvant une substance favorisant la circulation sanguine, une substance abaissant la pression sanguine, une substance favorisant le métabolisme dans la peau et une substance anti-inflammatoire,
**caractérisé en ce que** la biotine et/ou la tyrosine sont dissoutes en tant que la substance favorisant le métabolisme dans la peau, et le produit contient du poivre de Cayenne comme substance favorisant la circulation sanguine à une concentration de 2 à 10 mg/l.

15. Procédé selon la revendication 14,
**caractérisé en ce que** les substances mentionnées ci-après sont dissoutes dans le solvant, aux concentrations suivantes en mg/l :
| | |
|---|---|
| - nicotinate de benzyle : | 100,0 à 200,0 |
| - minoxidil : | 50,0 à 200,0 |
| - biotine : | 0,2 à 0,8 |
| - tyrosine : | 300,0 à 800,0 |
| - salicylate d'hydroxyéthyle : | 100,0 à 200,0 |
| - bituminosulfonate de sodium : | 3,0 à 15,0 |
